# EUROPEAN PATENT APPLICATION

(11) **EP 4 803 132 A1**
(43) Date of publication of application: **09.09.2026**
(21) Application number: 24885234.5
(22) Date of filing: 31.07.2024
(51) Int. Cl.: A61N 1/30, A61M 37/00

(54) **FLUID INJECTION DEVICE**

(30) Priority: 31.10.2023 JP 2023187013
(71) Applicant: Tohoku University, Sendai-shi, Miyagi 980-8577 (JP)
(72) Inventor: NISHIZAWA Matsuhiko, Sendai-shi, Miyagi 980-8577 (JP)
(74) Representative: Plougmann Vingtoft a/s
(86) International application number: PCT/JP2024/027334
(87) International publication number: WO 2025/094457

(57) **Abstract**

[Problem] To provide a fluid injection device capable of efficiently administering a drug.

[Solution] A first transport body 11 includes a first transport flow path through which a first fluid 21 flows, and a second transport body 12 includes a second transport flow path through which a second fluid 22 flows. A positive-side microneedle 13 has a first opening and a first flow path having a fixed positive charge, and is provided in the first transport body 11. A negative-side microneedle 14 has a second opening and a second flow path having a fixed negative charge, and is provided in the second transport body 12. The current/voltage application means 15 is configured so as to be capable of applying a current or voltage between the first electrode 24 disposed in the first transport flow path and the second electrode 25 disposed in the second transport flow path. When a current or voltage is applied, an ionic current flows through the first fluid 21 and the second fluid 22, and due to electroosmotic flow, the first fluid 21 flows outward from the first opening, and the second fluid 22 flows outward from the second opening.

## Description

### Field of the Invention

The present invention relates to a fluid injection device.

### Description of Related Art

Iontophoresis has conventionally been used for a drug administration method for promoting drug penetration performed by flowing a minute electric current through a living body. Since the iontophoresis is capable of administering drugs directly into blood vessel or an affected area, it has the advantage of allowing drugs to be administered more efficiently and minimizing side reactions compared to conventional oral administration.

The iontophoresis has widely been recognized to be particularly effective in promoting penetration of drugs through the skin (refer to, for example, Non-Patent Literature 1), and therefore various devices have been developed, ranging from stationary types that require an external power source to portable types and patch types to be to adhered to body surfaces. On the other hand, the Iontophoresis is also considered to be effective for administering medication to organs and tumors in addition to the skin, and it has been reported that it can significantly improve the efficiency of administering anticancer agents to cancer tumors (see, for example, Non-Patent Literature 2).

Such a penetration-promoting effect of the iontophoresis is considered to be realized not only by electrophoresis of drugs but also by "electroosmotic flow "occurring in skins and tissues. Since drug molecules are small in amount compared to electrolyte ions, it is considered that their movement by the electrophoresis is very small and in many cases the movement by electroosmotic flow dominates the whole. However, since skin and tissues in the body carry a slight negative charge, electroosmotic flow occurs only in a direction of cation migration. Therefore, the penetration is promoted only at an anode, and at a cathode, a flow occurs in the opposite direction, drawing out body fluids and potentially hindering drug penetration.

The present inventors have developed porous microneedles with fixed negative charge by chemical modification to generate large electroosmotic flows on the anode side (see, for example, Non-Patent Literatures 3 and 4, or Patent Literature 1). It has been reported that this microneedle makes it possible to penetrate drugs beneath the skin regardless of the electric charge carried by the drug, i.e., regardless of the direction of electrophoresis.

In response, the present inventors have developed an electroosmotic flow pump using a material with a fixed negative charge on the anode side and a material with a fixed positive charge on the cathode side, capable of supplying drugs to the surface of the skin or tissue using electroosmotic flow from both the anode and cathode sides without interfering with drug penetration on the cathode side (see, for example, Patent Literature 2).

### Citation List

### Non-Patent Literature

Non-Patent Literature 1: Kenji Sugibayashi, "Iontophoresis, New Transdermal Administration Method", Farumashia, 2001, Vol. 37, No. 5, pp. 385-387
Non-Patent Literature 2: James D. Byrne et al., "Local iontophoretic administration of cytotoxic therapies to solid tumors", Science Translational Medicine, 2015, 7, 273ra14
Non-Patent Literature 3: Shinya Kusama et al., "Transdermal Electroosmotic Flow Generated by a Porous Microneedle Array Patch", Nature Communications, 2021, 12, 658
Non-Patent Literature 4: Hiroya Abe et al., "Porous Microneedle Patch for Electroosmosis-Promoted Transdermal Delivery of Drugs and Vaccines", Advanced NanoBiomedical Research, 2022, 2, 2100066

### Patent Literature

Patent Literature 1: JP 2022-083780 A
Patent Literature 2: JP 2023-069170 A

### Summary of the Invention

### [Problems to be solved by the invention]

The electroosmotic flow pump disclosed in Patent Literature 2 is capable of supplying a drug to the surface of the skin or tissue from both the anode and cathode sides, thereby increasing the amount of drug to be administered. Moreover, it is possible to simultaneously administer two kinds of drugs by supplying different drugs respectively from the anode side and the cathode side. As described above, the electroosmotic flow pump disclosed in Patent Literature 2 makes it possible to administer drugs more efficiently than devices using only electroosmotic flow on the anode side and the cathode side, and therefore it is expected that devices capable of administering drugs even more efficiently is developed.

The present invention has been made with a focus on these problems and aims to provide a fluid injection device capable of more effectively administering a drug.

### [Means for solving the problems]

In order to achieve the above mentioned object, a fluid injection device according to the present invention includes: a first transport body including a first transport flow path, a first fluid flowing through the first transport flow path; a second transport body including a second transport flow path, a second fluid flowing through the second transport flow path; a positive-side microneedle provided in the first transport body, the positive-side microneedle including a first opening at a tip thereof and a first flow path having a fixed positive charge, the first flow path communicating with the first opening, so that the first flow path communicates with the first transport flow path; a negative-side microneedle provided in the second transport body, the negative-side microneedle including a second opening at a tip thereof and a second flow path having a fixed negative charge, the second flow path communicating with the second opening, so that the second flow path communicates with the second transport flow path; and a current/voltage application means including a first electrode disposed in the first transport flow path and a second electrode disposed in the second transport flow path, the current/voltage application means capable of applying a current or voltage between the first electrode and the second electrode, wherein when the current/voltage application means applies a current or voltage between the first electrode and the second electrode, an ionic current flows through the first fluid in the first flow path and the second fluid in the second flow path, and due to electroosmotic flow, the first fluid flows outward from the first opening and the second fluid flows outward from the second opening.

The fluid injection device according to the present invention is configured so that a positive-side microneedle is provided on the first transport body and a negative-side microneedle is provided on the second transport body, and the first fluid flows outward from the first opening of the positive-side microneedle and the second fluid flows outward from the second opening of the negative-side microneedle due to the electroosmotic flow, thereby making it possible to directly inject the first fluid and the second fluid subcutaneously or into the tissue. Therefore, by using fluids containing a drug as the first fluid and the second fluid, it is possible to administer the drug more efficiently compared to conventional electroosmotic pumps with no microneedle.

When the fluid injection device according the present invention uses fluids containing a drug as the first fluid and the second fluid, it is possible to supply the drug subcutaneously or into the tissue from both the first opening of the positive-side microneedle and the second opening of the negative-side microneedle, thereby increasing the amount of drug administered by up to two times compared to supplying the drug from only any one of the openings. Moreover, by using different drugs respectively for the drug contained in the first fluid and the drug contained in the second fluid, it is possible to simultaneously administer two types of drugs.

The fluid injection device according to the present invention applies the current or voltage to the first electrode, which is negative, and the second electrode, which is positive, using the current/voltage application means, thereby making it possible to flow the ionic current through the first fluid in the first flow path and the second fluid in the second flow path, and due to electroosmotic flow, to flow the first fluid outward from the first opening and to flow the second fluid outward from the second opening. The fluid injection device according to the present invention is capable of controlling a flow rate of the generated electroosmotic flow by adjusting the current or voltage applied by the current/voltage application means, and has excellent controllability over the discharge amounts of the first fluid and the second fluid.

In the fluid injection device according to the present invention, the positive-side microneedle and the negative-side microneedle may be made of a porous body, and the first flow path and the second flow path may respectively extend in a net shape through voids in the porous body. Moreover, each of the positive-side microneedle and the negative-side microneedle may be composed of one microneedle or may be composed of a plurality of microneedles. When the positive-side and negative-side microneedles are composed of a plurality of microneedles, each may form a microneedle array. Moreover, the positive-side microneedle and the negative-side microneedle may each have a sharp tip so as to be inserted into a object such as skin or tissue to directly inject the first fluid and the second fluid into the object, or they may each have a curved or flat tip so as to be spread the surface of the object and allow the first fluid and the second fluid to penetrate the object. Moreover, each of the first opening and the second opening may be composed of one opening or may be composed of a plurality of openings.

In the fluid injection device according to the present invention, the positive-side microneedle may be made of any material capable of fixing a positive charge in the first flow path, and the negative-side microneedle may be made of any material capable of fixing a negative charge in the second flow path. In this case, the positive-side microneedle and the negative-side microneedle are made of, for example, a hydrogel material, a porous resin, an oxide, a metal, a biodegradable material, or the like. More specifically, the positive-side microneedle may have a greater mobility of anions than that of cations when a fluid is introduced into the first flow path; for example, a positive charge may be fixed to a wall of the first flow path, a positive charge may be embedded in a surface layer thereof, or the positive-side microneedle may be made of a hydrogel containing a functional group having a positive charge. Moreover, the negative-side microneedle may have a greater mobility of cations than that of anions when a fluid is introduced into the second flow path; for example, a negative charge may be fixed to a wall of the second flow path, a negative charge may be embedded in a surface layer thereof, or the negative-side microneedle may be made of a hydrogel containing a functional group having a negative charge.

In the fluid injection device according to the present invention, the first fluid and the second fluid are preferably fluids each containing a drug, and may be made of different fluids or the same fluid. In the fluid injection device of the present invention, it is preferable that the first electrode is inserted into the first transport flow path from the other end side of the first transport body, and the second electrode is inserted into the second transport flow path from the other end side of the second transport body.

n the fluid injection device according to the present invention, it is preferable that the positive-side microneedle and the negative-side microneedle are disposed adjacent to each other. In this case, the entire device can be configured compactly, and miniaturization can be realized. Moreover, in this case, it is preferable that the tips of the positive-side and negative-side microneedles protrude on the same side so as to be easily inserted into or pressed against an object at the same time.

In the fluid injection device according to the present invention, the first transport body may be elongated and the first transport flow path may extend from one end to the other end, the second transport body may be elongated and the second transport flow path may extend from one end to the other end, the positive-side microneedle may be provided at the one end of the first transport body, and the negative-side microneedle may be provided at the one end of the second transport body. In this case, the entire device can be made elongated, and miniaturization can be realized. In this case, it is also preferable that the positive-side microneedle is disposed so that its tip protrudes from the one end of the first transport body along an extension direction of the first transport body, and the negative-side microneedle is disposed so that its tip protrudes from the one end of the second transport body along an extension direction of the second transport body. This makes it possible for the positive-side and negative-side microneedles to be easily inserted into or pressed against the object by moving one end of the first transport body and one end of the second transport body toward the object respectively along their extension directions.

In the fluid injection device according to the present invention, the first transport body and the second transport body may be formed separately from each other or may be formed integrally. When the fluid injection device is formed integrally, the entire device can be made compact, miniaturized, and easier to handle.

The fluid injection device according to the present invention may include a rigid tubular body integrally forming the first transport body and the second transport body, the tubular body may be elongated and may have two hollow portions therein extending from one end to the other, one of the hollow portions may form the first transport flow path, and the other hollow portion may form the second transport flow path. In this case, the tubular body can be held in one hand, allowing the positive-side microneedle and negative-side microneedle to be easily inserted into or easily pressed against the object.

Moreover, in the fluid injection device according to the present invention, the first transport body and the second transport body may each be made of a flexible tube. In this case, for example, by using a catheter as the tube, it is possible to easily access the tissue inside the living body and to directly inject a drug or the like into the tissue. This makes it possible to directly administer, for example, anticancer drugs to tumors, thereby providing effective treatment.

### [Advantageous effect of the invention]

In accordance with the present invention, it is possible to provide the fluid injection device capable of more effectively administering a drug.

### Brief Description of the Drawings

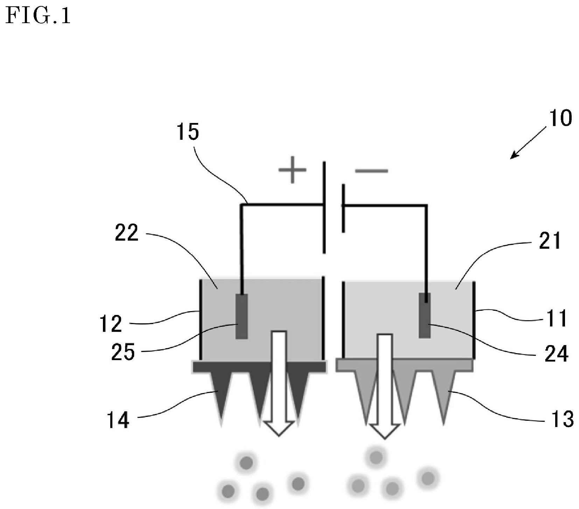

FIG. 1 is cross-sectional diagram showing a fluid injection device according to an embodiment of the invention.

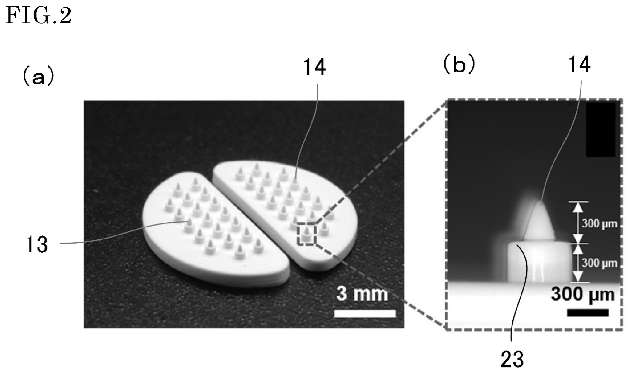

FIG. 2(a) is a perspective view showing an array of positive-side and negative-side microneedles and FIG. 2(b) is an enlarged side view of one negative-side microneedle, in a fluid injection device according to an embodiment of the present invention.

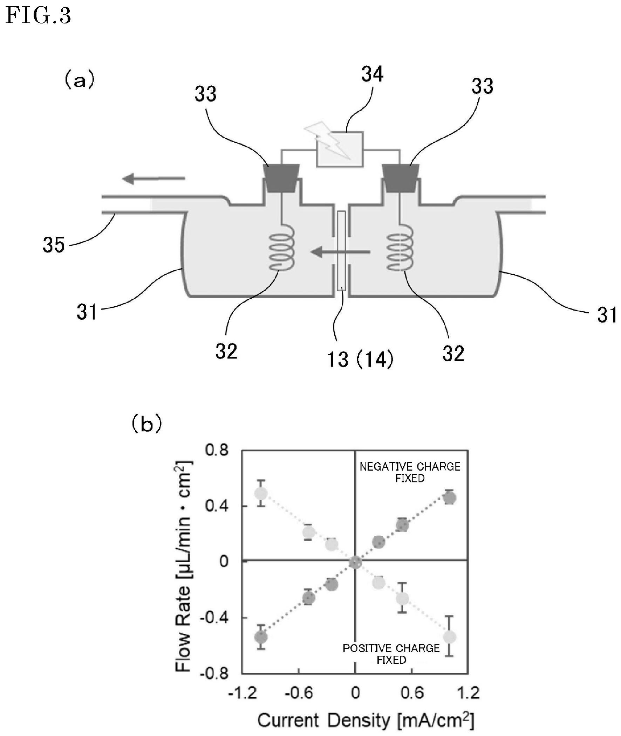

FIG. 3(a) is a cross-sectional view showing a test method for an electroosmotic flow generation test using a Franz cell with a horizontal capillary with respect to the positive-side microneedle and the negative-side microneedle of a fluid injection device according to an embodiment of the present invention, and FIG. 3(b) is a graph showing a test result, i.e., a relationship between current density and flow rate for a positive-side microneedle (positive charge fixed shown in the figure) and a negative-side microneedle (negative charge fixed shown in the figure).

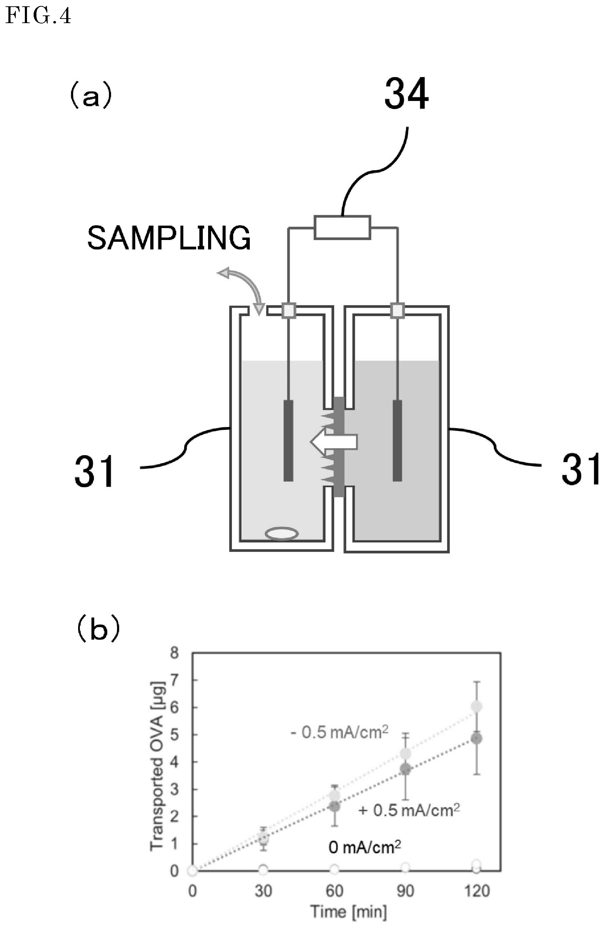

FIG. 4(a) is a cross-sectional view showing the a method, for an electroosmotic flow generation test using a Franz cell with respect to the positive-side and negative-side microneedles of a fluid injection device according to an embodiment of the present invention, and FIG. 4(b) is a graph showing a change over time in the amount of FITC-OVA molecular transport (Transported OVA) for a positive-side microneedle ("-0.5 mA/cm²" in the figure) and a negative-side microneedle ("0.5 mA/cm²" in the figure).

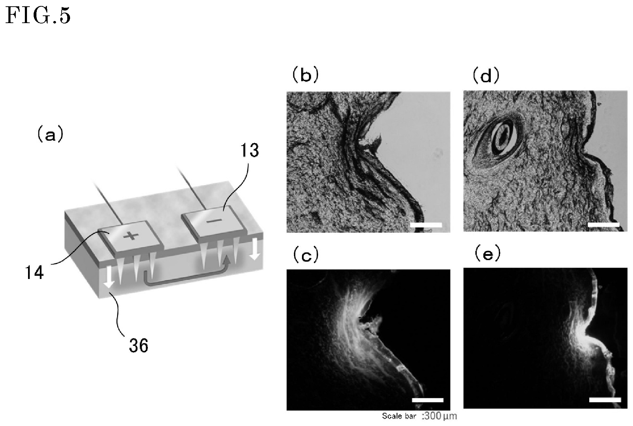

FIG. 5(a) is a perspective view showing a test method for injecting dextran into a pig skin slice using a fluid injection device according to an embodiment of the present invention; FIG. 5(b) is a micrograph of the pig skin slice taken under bright light when 0.5 mA/cm² is applied to the negative-side microneedle, and FIG. 5(c) is a micrograph taken under fluorescent light; and FIG. 5(d) is a micrograph of the pig skin slice taken under bright light when -0.5 mA/cm² is applied to the positive-side microneedle, and FIG. 5(e) is a micrograph taken under fluorescent light.

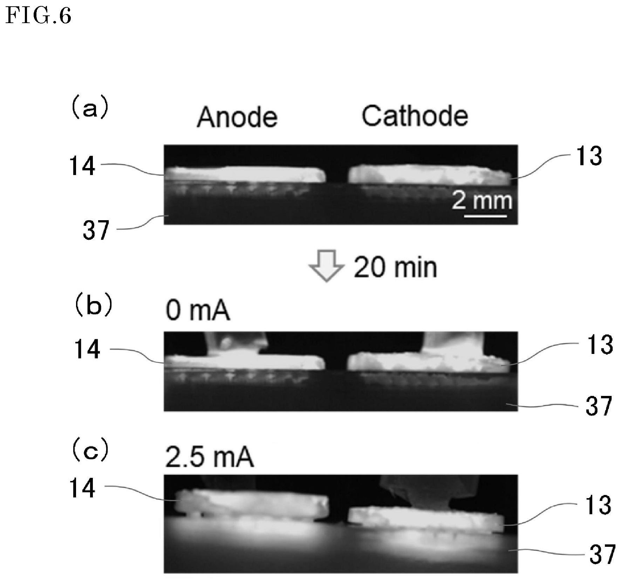

FIG. 6(a) is a side view showing fluorescence due to dextran in a gellan gum gel before a test injecting dextran into the gellan gel using a fluid injection device according to an embodiment of the present invention; FIG. 6(b) is a side view showing the fluorescence due to dextran in the gellan gum gel after the device has been left for 20 minutes without applying electric current (0 mA); and FIG. 6(c) is a side view showing the fluorescence due to dextran in the gellan gum gel after electric current of 2.5 mA is applied between the anode side on the positive-side microneedle side and the cathode side on the negative-side microneedle side for 20 minutes.

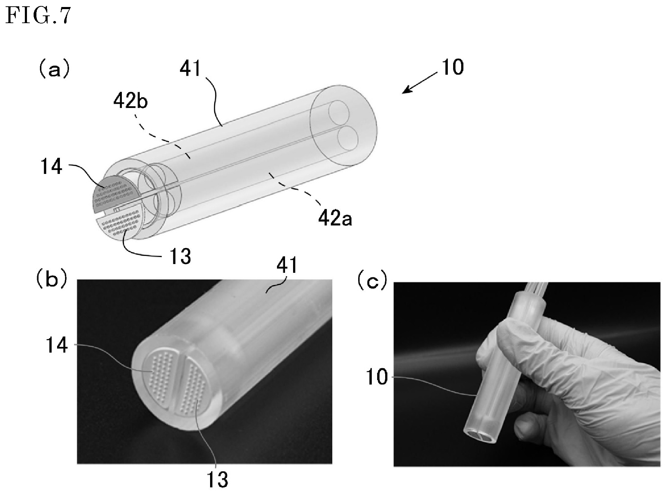

FIG. 7(a) is an exploded perspective view showing an example of a stamp-type device of a fluid injection device according to an embodiment of the present invention, FIG. 7(b) is an enlarged perspective view of one end of the tubular body, and FIG. 7(c) is a perspective view showing the device held in one hand during use.

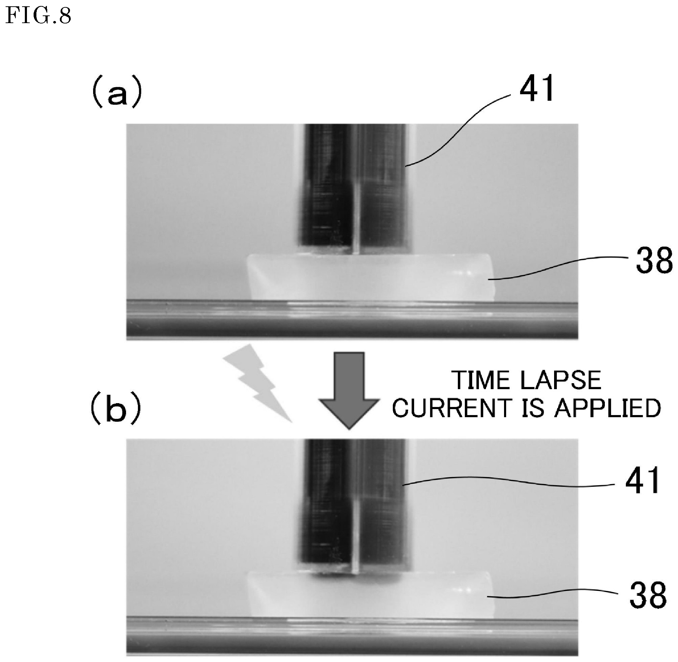

FIG. 8(a) is a side view of the gellan gum gel before a test for injecting rhodamine B and methylene blue into a gellan gum gel using the stamp-type device shown in FIG. 7, and FIG. 8(b) is a side view showing the penetration of rhodamine B and methylene blue into the gellan gum gel when a current of 2.0 mA is applied between the first electrode and the second electrode for 20 minutes.

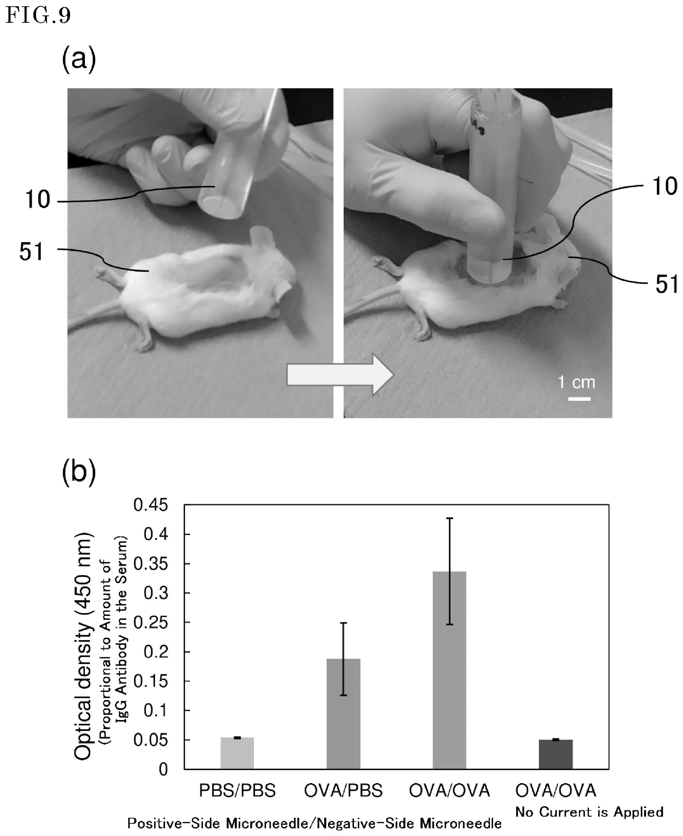

FIG. 9(a) is a perspective view showing a state before and during fluid injection in a vaccination test of a vaccine model (OVA) into mice using the stamp-type device shown in FIG. 7, and FIG. 9(b) is a graph showing absorbance (optical density) for each combination of positive-side and negative-side microneedles.

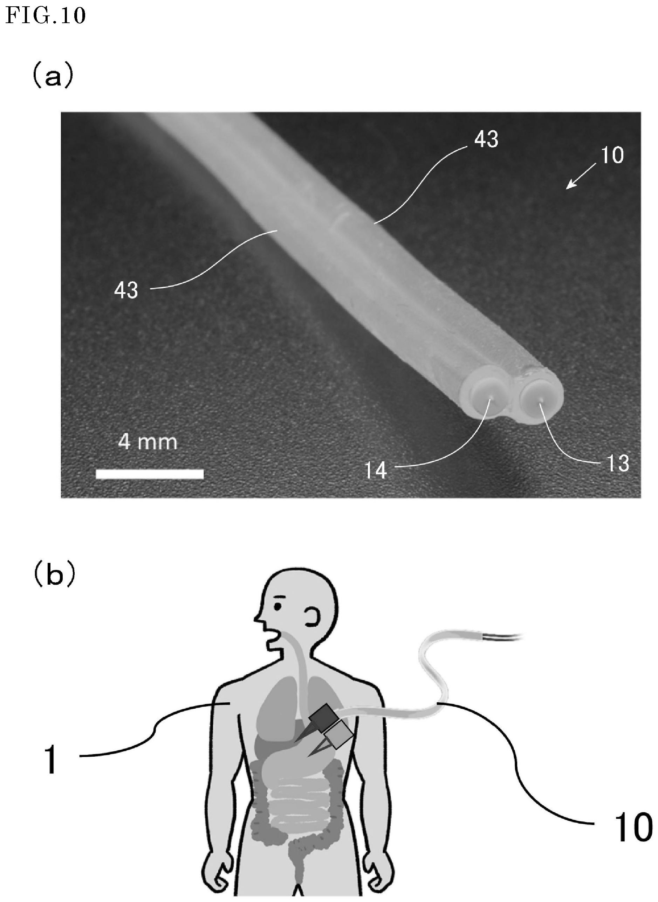

FIG. 10(a) is an enlarged perspective view of one end of a tube showing an example of a catheter-type fluid injection device according to an embodiment of the present invention, and FIG. 10(b) is a side view showing the device in use.

### Detailed Description of the Invention

Embodiments of the present invention will be described with reference to the drawings, hereinafter.

FIGS. 1 to 10 show a fluid injection device according to an embodiment of the present invention.

As shown in FIG. 1, the fluid injection device 10 includes a first transport body 11, a second transport body 12, a positive-side microneedle 13, a negative-side microneedle 14, and a current/voltage application means 15.

The first transport body 11 is elongated and has a first transport flow path (not shown) extending from one end to the other end inside. The first transport body 11 is configured to flow a first fluid 21 through the first transport flow path. The second transport body 12 is elongated and has a second transport flow path (not shown) extending from one end to the other end inside. The second transport body 12 is configured to flow a second fluid 22 through the second transport flow path. The first transport body 11 and the second transport body 12 may be configured separately from each other or may be formed integrally.

The first fluid 21 flowing through the first transport flow path and the second fluid 22 flowing through the second transport flow path may be any fluid suitable for injection into an object, but when the object is skin or tissue inside a living body, it is preferable that the fluids contain a drug. The first fluid 21 and the second fluid 22 may be made of different fluids or may be made of the same fluid.

As shown in FIG. 2, the positive-side microneedle 13 and the negative-side microneedle 14 are each made of a porous body, have a conical shape, and have a flange portion 23 around the rear end portion on the conic base side. The positive-side microneedle 13 includes at least a first opening (not shown) provided at a tip thereof, and a first flow path (not shown) having a fixed positive charge and communicating with the first opening. The negative-side microneedle 14 includes at least a second opening (not shown) provided at a tip thereof, and a second flow path (not shown) having a fixed negative charge and communicating with the second opening. The first flow path and the second flow path extend in a net shape through void portions in the porous body. In a specific example shown in FIG. 2, the height of the tip of each of the positive-side microneedle 13 and the negative-side microneedle 14 is 300 µm, and the height of the flange portion 23 thereof is 300 µm.

As shown in FIG. 1, the positive-side microneedle 13 is provided at one end of the first transport body 11 so that the first flow path communicates with the first transport flow path. Moreover, positive-side microneedle 13 is provided so that its tip protrudes from one end of the first transport body 11 along the extension direction of the first transport body 11. The negative-side microneedle 14 is provided at one end of the second transport body 12 so that the second flow path communicates with the second transport flow path. Moreover, negative-side microneedle 14 is provided so that its tip protrudes from one end of the second transport body 12 along the extension direction of the second transport body 12. Furthermore, the positive-side microneedle 13 and the negative-side microneedle 14 are disposed adjacent to each other so that each tip protrudes on the same side.

The positive-side microneedle 13 and the negative-side microneedle 14 may each be composed of a plurality of microneedles as shown in FIG. 2, or may each be composed of one microneedle. When the positive-side and negative-side microneedles are composed of a plurality of microneedles, each may form a microneedle array. Moreover, the positive-side microneedle 13 and the negative-side microneedle 14 may each have a sharp tip so as to be inserted into a object to directly inject the first fluid 21 and the second fluid 22 into the object, or they may each have a curved or flat tip so as to be spread the surface of the object and allow the first fluid 21 and the second fluid 22 to penetrate the object. Moreover, each of the first opening and the second opening may be composed of one opening or may be composed of a plurality of openings.

The positive-side microneedle 13 may be made of any material capable of fixing a positive charge in the first flow path, and the negative-side microneedle 14 may be made of any material capable of fixing a negative charge in the second flow path. The positive-side microneedle 13 and the negative-side microneedle 14 are made of, for example, a hydrogel material, a porous resin, an oxide, a metal, a biodegradable material, or the like. More specifically, the positive-side microneedle 13 may have a greater mobility of anions than that of cations when a fluid is introduced into the first flow path; for example, a positive charge may be fixed to a wall of the first flow path, a positive charge may be embedded in a surface layer thereof, or the positive-side microneedle may be made of a hydrogel containing a functional group having a positive charge. Moreover, the negative-side microneedle 14 may have a greater mobility of cations than that of anions when a fluid is introduced into the second flow path; for example, a negative charge may be fixed to a wall of the second flow path, a negative charge may be embedded in a surface layer thereof, or the negative-side microneedle may be made of a hydrogel containing a functional group having a negative charge.

The hydrogel material herein refers to the material that forms hydrogel by being dispersed in water (dispersion medium). Examples of the hydrogel material include natural polymers of agar, gelatin, agarose, xanthan gum, gellan gum, sclerotium gum, gum arabic, gum tragacanth, gum karaya, cellulose gum, tamarind gum, guar gum, locust bean gum, glucomannan, chitosan, carrageenan, quince seed, galactan, mannan, starch, dextrin, curdlan, casein, pectin, collagen, fibrin, peptide, chondroitin sulfate such as chondroitin sulfate sodium salt, hyaluronate such as hyaluronic acid (mucopolysaccharide) and sodium hyaluronate, alginate such as alginic acid, sodium alginate and calcium alginate, and their derivatives; cellulose derivatives of methylcellulose, hydroxymethyl cellulose, hydroxyethyl cellulose, hydroxypropyl cellulose, hydroxypropyl methylcellulose, carboxymethyl cellulose, and their salts; poly (meth) acrylic acids of polyacrylic acid, polymethacrylic acid, sodium polymethacrylate, and acrylic acid-alkyl methacrylic acid copolymer, and their salts; synthetic polymers of polyvinyl alcohol, polyhydroxyethyl methacrylate, polyacrylamide, poly (N-isopropyl acrylamide), polyvinylpyrrolidone, polystyrene sulfonate, polyethylene glycol, carboxyvinyl polymer, alkyl modified carboxyvinyl polymer, maleic anhydride copolymer, polyalkylene oxide resin, crosslinked product of poly (methyl vinyl ether-alt-maleic anhydride) and polyethylene glycol, polyethylene glycol crosslinked product, N-vinyl acetamide crosslinked product, acrylamide crosslinked product, and crosslinked product of starch-acrylate graft copolymer; silicone; interpenetrating network hydrogel and semi-interpenetrating network hydrogel; poly 2-hydroxyethyl methacrylic acid, and poly 2-acrylamide-2-methyl propane sulfonic acid; and mixtures of two or more materials among these. Preferable hydrogel materials among these materials, from the viewpoint of withstand load and bioaffinity, are collagen, and glucomannan; carboxymethyl cellulose, and sodium carboxymethyl cellulose; polyacrylic acid, and sodium polyacrylate; and interpenetrating network hydrogel, and semi-interpenetrating network hydrogel. A preferable hydrogel material, from the viewpoint of excellent mechanical strength and excellent biocompatibility, is a crosslinked product of poly (methyl vinyl ether-alt-maleic anhydride) and polyethylene glycol. Moreover, a preferable hydrogel material, from the viewpoint of ensuring electrical neutrality in hydrogel, is crosslinked polyethylene glycol.

Moreover, examples of hydrogel materials having a fixed charge (positive or negative charge) include gel materials in which functional groups having a fixed charge is introduced into hydrogel materials having no fixed charge, and gel materials that are macromolecules (polymers) containing monomer units having a fixed charge. Among these, gel materials that are macromolecules (polymers) containing monomer units having a fixed charge are preferred, and copolymers of a non-charged monomer and a monomer having a fixed charge are more preferred.

Examples of the resin include polycarbonate, acrylonitrile butadiene styrene (ABS) resin, phenolic resin, acrylic resin, methacrylic resin (such as, polyglycidyl methacrylate resin). Examples of the oxide include inorganic oxide and its derivative. Examples of the inorganic oxide herein include silicon oxide, tin oxide, zirconia oxide, titanium oxide, niobium oxide, tantalum oxide, aluminum oxide, tungsten oxide, hafnium oxide, and zinc oxide. Examples of the metal include nickel, iron, and these alloy. Examples of the biodegradable material include poly lactic-co-glycolic acid (PLGA) and mixed material mainly made of PLGA, β-tricalcium phosphate, calcium carbonate, polycaprolactone, polydioxanone, hydroxyapatite, polyethylene glycol, and magnesium alloy. The positive-side microneedle 13 and the negative-side microneedle 14 may be made of combination of two or more of the substances indicated above.

The current/voltage application means 15 has a first electrode 24 disposed in the first transport flow path and a second electrode 25 disposed in the second transport flow path, and is configured so as to be capable of applying a current or voltage between the first electrode 24 and the second electrode 25. The first electrode 24 is inserted into the first transport flow path from the other end side of the first transport body 11, and the second electrode 25 is inserted into the second transport flow path from the other end side of the second transport body 12. More specifically, the current/voltage applying means 15 is configured to apply a current or voltage with the first electrode 24 as a cathode and the second electrode 25 as an anode. As a result, the fluid injection device 10 is configured so that when the current/voltage application means 15 applies a current or voltage between the first electrode 24 and the second electrode 25, an ionic current flows through the first fluid 21 in the first flow path and the second fluid 22 in the second flow path, and due to electroosmotic flow, the first fluid 21 flows outward from the first opening and the second fluid 22 flows outward from the second opening.

Next, effective action will be described.

The fluid injection device 10 is used as follows. First, the positive-side microneedle 13 provided at one end of the first transport body 11 and the negative-side microneedle 14 provided at one end of the second transport body 12 are inserted into or pressed against the skin or tissue inside a living body. In this state, a current or voltage is applied between the first electrode 24 and the second electrode 25 by the current/voltage application means 15. This makes it possible to flow the ionic current through the first fluid 21 in the first flow path and the second fluid 22 in the second flow path, and due to electroosmotic flow, to flow the first fluid 21 outward from the first opening of the positive-side microneedle 13 and to flow the second fluid 22 outward from the second opening of the negative-side microneedle 14. This makes it possible for the fluid injection device 10 to inject first fluid 21 and second fluid 22 directly subcutaneously or into the tissue. Therefore, by using fluids containing a drug as the first fluid 21 and the second fluid 22, it is possible to administer the drug more efficiently compared to conventional electroosmotic pumps with no microneedle.

Moreover, when the fluid injection device 10 uses fluids containing a drug as the first fluid 21 and the second fluid 22, it is possible to supply the drug subcutaneously or into the tissue from both the first opening of the positive-side microneedle 13 and the second opening of the negative-side microneedle 14, thereby increasing the amount of drug administered by up to two times compared to supplying the drug from only any one of the openings. Moreover, by using different drugs respectively for the drug contained in the first fluid 21 and the drug contained in the second fluid 22, it is possible to simultaneously administer two types of drugs.

The fluid injection device 10 is capable of controlling a flow rate of the generated electroosmotic flow by adjusting the current or voltage applied by the current/voltage application means 15, and has excellent controllability over the discharge amounts of the first fluid 21 and the second fluid 22. Moreover, in the fluid injection device 10, the positive-side microneedle 13 and the negative-side microneedle 14 are adjacent to each other and are disposed so that their tips protrude to the same side, allowing the entire device to be configured compactly and miniaturized. Moreover, the positive-side microneedles 13 and the negative-side microneedles 14 can be easily inserted or pressed against an object at the same time, making them easy to handle.

### [Example 1]

The positive-side microneedle 13 and the negative-side microneedle 14 of the fluid injection device 10 are produced, and the fluid injection device 10 is subjected to various tests. The reagents and the materials used in the production and the tests are as follows:

Glycidyl Methacrylate (GMA), made by FUJIFILM Wako Pure Chemical Corporation
Trimethylolpropane trimethacrylate (TRIM), made by Sigma-Aldrich Co. LLC
Polyethylene glycol (PEG) 10 kDa, made by Sigma-Aldrich Co. LLC
Diethylene glycol (DEG), made by Tokyo Chemical Industry Co., Ltd.
Irgacure 184, made by BASF SE
Polydimethylsiloxane (PDMS), SILPOT 184, made by DuPont Toray Specialty Materials K.K.
Gellan gum, made by FUJIFILM Wako Pure Chemical Corporation
Triethylene glycol dimethacrylate (TEGDMA)
(3-acrylamidopropyl) trimethylammonium (APTA)
2-acrylamide-2-methylpropanesulfonic acid (AMPS)
2,2'-Azobis[2-(2-imidazolin-2-yl)propane]dihydrochloride (VA-044)
N,N'-methylenebisacrylamide (MBAAmM)
N,N,N',N'-tetramethylethylenediamine (TEMED)
Ammonium peroxodisulfate solution (APS), 10w/v%
Fluorescein isothiocyanate-dextran (FITC dextran), average mol wt 500.000, made by Sigma-Aldrich Co. LLC
Fluorescein isothiocyanate-ovalbumin (FITC-OVA), 4.4kDa, 0.75 mg/mL, made by Sigma-Aldrich Co. LLC
1xPBS(-) (phosphate buffered saline), made by FUJIFILM Wako Pure Chemical Corporation

### [Production of microneedle array]

An array of the positive-side microneedles 13 and the negative-side microneedles 14 is produced. First, an acrylic board is drilled using a cutting machine to create a female mold for the protrusions of each microneedle. The female mold is transferred in two stages by use of the PDMS, and a female mold made of the PDMS is produced. Subsequently, a precursor solution for forming a porous material body is prepared. The precursor solution is prepared by mixing a photoinitiator in a solution A and a solution B, each having the following composition. The solution A is prepared by mixing the PEG (4 g), which can form pores in the structure when eluted, and the DEG (20 mL) of a solvent at 60°C. The solution B is prepared by mixing the GMA (10 mL) of a monomer, and the TRIM (5.23 mL) and TEGDMA (15.7 mL) of cross-linking agents.

The precursor solution is then obtained by mixing the solution A (450 µL), the solution B (550 µL), and photoinitiator Irgacure 184 (1.8 mg) at 40°C. The precursor solution is poured in the female mold made of the PDMS, and is subjected to degassing at 25°C for 80 minutes under a reduced pressure of -0.096 MPa. The degassing process enables to prevent a defect in shape of the needle from occurring due to mixed bubbles. After the degassing process, it is exposed to irradiation of ultraviolet rays with a wavelength of 365 nm at 25°C for one hour, so that the monomer and the cross-linking agent are polymerized and solidified. Then, a solidified protrusion array substrate is taken out from the female mold. Then, the protrusion array substrate is immersed in a mixed solution of distilled water and the methanol overnight (1:1 in volume ratio), to elute the PEG. In this way, a microneedle array having a porous structure as shown in FIG. 2 was produced.

Next, the produced porous microneedle array is immersed in a silane coupling agent solution (2 mL; TMSPMA 0.6 mL, ethanol 1.4 mL) at room temperature for one hour to modify a surface including the inside of the pores, with the silane coupling agent. Then, the substrate modified with the silane coupling agent is rinsed twice in distilled water for 15 minutes each while being shaken using a shaker to remove excess unreacted TMSPMA and ethanol. After thoroughly wiping off the moisture, the substrate is immersed in the following solution (1) for fixing a negative charge, or in the following solution (2) for fixing a positive charge, at 4°C for 8 hours:
(1) 0.05 M AMPS, 1 v% APS and 0.1 v% TEMED
(2) 0.4 M APTAC with 2 v% VA-044

After immersion in each solution, polymerization is carried out in an oven at 80°C for one hour, whereby the pore walls of the microneedle array are modified with AMPS or PAPTAC by graft polymerization. In this way, an array of negative-side microneedles 14 having a fixed negative charge and an array of positive-side microneedles 13 having a fixed positive charge are produced.

### [Generation test of electroosmotic flow]

A generation test of electroosmotic flow is conducted using the produced array of positive-side microneedles 13 and negative-side microneedles 14. First, a test is conducted using a Franz cell with a horizontal capillary as shown in FIG. 3(a). In the test, the positive-side microneedle 13 or the negative-side microneedle 14 is sandwiched between the two chamber 31 of the Franz cell to be fixed with the jig made of an acrylic board. Both chambers 31 (opening diameter 15mm) of the Franz cell are filled with a PBS buffer solution of pH 7.0, and an injection port of each chamber 31 is closed with a silicone rubber stopper 33 inserted with an Ag/Cl wire 32.

In this state, the Ag/Cl wire 32 (AgCl + e- 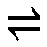 Ag + Cl-) is connected to a source meter 34, and by quarter applied -1.0, -0.5, -0.25, 0, 0.25, 0.5, direct current of 1.0mA/cm² and measured aged deterioration of the water surface position in horizontal capillary 35 (cross-sectional diameter 1.6mm) using a camera every 5 minutes. During the test, it is confirmed that no bubbles are generated from the Ag/Cl wire 32 due to the electrode reaction.

The test results are shown in FIG. 3(b). As shown in FIG. 3(b), it is confirmed that electroosmotic flow is generated in both the positive-side microneedle 13 (corresponding to the "positive charge fixed" shown in the figure) and the negative-side microneedle 14 (corresponding to the "negative charge fixed" shown in the figure), and the flow rate increases as the current density increases. Moreover, it is also confirmed that the flow directions are opposite to each other between the positive-side microneedle 13 and the negative-side microneedle 14, and the slopes of the respective graphs (electroosmotic flow efficiency) are approximately the same.

Next, using the Franz cell shown in FIG. 4(a), a test is conducted so that a mixture of FITC-OVA and PBS buffer solution is introduced into both chambers 31 to measure the amount of molecular transport die to electroosmotic flow. In the test, a direct current of -0.5 mA/cm² (for the positive-side microneedle 13) or 0.5 mA/cm² (for the negative-side microneedle 14) is applied between each cell from the source meter 34, and every 30 minutes, a small amount of the solution in the receptor side chamber 31 (chamber 31 on the left side of the figure) is sampled and quantitatively analyzed using a plate reader to measure the amount of molecules transported from the donor side chamber 31 (chamber 31 on the right side of the figure) to the receptor side chamber 31.

The test results are shown in FIG. 4(b). As shown in FIG. 4(b), electroosmotic flow is generated in both the positive-side microneedle 13 (corresponding to the "-0.5 mA/cm²" in the figure) and the negative-side microneedle 14 (corresponding to the "0.5 mA/cm²" in the figure), and it is confirmed that the amount of FITC-OVA molecules transported (Transported OVA) is increased over time. Moreover, it is also confirmed that approximately the same amount of transport is generated in opposite directions between the positive-side microneedle 13 and the negative-side microneedle 14. In addition, measurements are also conducted without current flow (corresponding to the "0 mA/cm²" in the figure), and it is confirmed that no transport is generated.

Next, as shown in FIG. 5(a), a test is conducted to inject dextran into a pig skin slice 36 using the fluid injection device 10 shown in FIG. 1, which uses the produced positive-side microneedle 13 and negative-side microneedle 14. In the test, FITC dextran is used as the first fluid 21 and the second fluid 22, and the penetration state of FITC dextran into the inside of the pig skin slice 36 is observed using a fluorescence microscope when -0.5 mA/cm² is applied to the positive-side microneedle 13 and when 0.5 mA/cm² is applied to the negative-side microneedle 14.

The test results are shown in FIGS. 5(b) to 5(e). As shown in FIGS. 5(b) to 5(e), both when current is applied to the positive-side microneedle 13 and when current is applied to the negative-side microneedle 14, fluorescence is observed inside the pig skin slice 36, confirming that the FITC dextran penetrates the skin.

Next, a test is conducted to inject dextran into a gellan gum gel 37 using the fluid injection device 10 shown in FIG. 1, which uses the produced positive-side microneedle 13 and negative-side microneedle 14. In the test, agarose gel containing FITC dextran is used as the first fluid 21 and the second fluid 22, and the positive-side microneedle 13 and the negative-side microneedle 14 are inserted into the gellan gum gel 37, and electricity is passed through the negative-side microneedle 14 side, which is an anode, and the positive-side microneedle 13 side, which is a cathode.

The test results are shown in FIGS. 6(a) to 6(c). As shown in FIG. 6(b), when the device is left for 20 minutes without applying current (0 mA), no fluorescence was observed in the gellan gum gel 37, confirming that no FITC-dextran penetrates the gel. In contrast, as shown in FIG. 6(c), when a current of 2.5 mA is applied for 20 minutes, fluorescence is observed in the gellan gum gel 37, confirming that FITC dextran permeates the gel from both electrodes.

### [Example 2]

### [Example of fluid injection device: Stamp-type device]

As shown in FIG. 7, the fluid injection device 10 has a rigid tube 41 that integrally forms the first transport body 11 and the second transport body 12, and the tube 41 is elongated and has two hollow portions 42a, 42b extending from one end to the other end, one hollow portion 42a may form the first transport flow path and the other hollow portion 42b may form the second transport flow path.

In the example shown in FIG. 7, a fluid injection device 10 is structured such that a semicircular positive-side microneedle 13 and a semicircular negative-side microneedle 14 are fitted into one end of a tube 41 so that the first flow path of the positive-side microneedle 13 communicates with the first transport flow path and the second flow path of the negative-side microneedle 14 communicates with the second transport flow path. The tubular body 41 is made of resin and produced using a 3D printer.

As shown in FIG. 7( c ), the fluid injection device 10 is used to hold the tube 41 in one hand and to press one end of the tube 41 against the object. As a result, the fluid injection device 10 allows the positive-side microneedle 13 and negative-side microneedle 14 to be easily inserted into or easily pressed against the object. The fluid injection device 10 can be used, for example, on a body surface, scalp, and also on organs and tumors exposed by laparotomy. Moreover, since the fluid injection device 10 is integrally formed as a tubular body, the entire device can be configured compactly, thereby realizing miniaturization.

A fluid injection test is conducted using the fluid injection device 10 shown in FIG. 7. In the test, a solution of rhodamine B (0.75 mg/mL, 479 Da) is used as the first fluid 21 on the positive-side microneedle 13 side, and a solution of methylene blue (0.75 mg/mL, 320 Da) is used as the second fluid 22 on the negative-side microneedle 14 side. As shown in FIG. 8(a), the positive-side microneedle 13 and the negative-side microneedle 14 are inserted into the gellan gum gel 38, and a current of 2.0 mA is applied between the first electrode 24 and the second electrode 25 for 20 minutes. The result is shown in FIG. 8. As shown in FIG. 8(b), it is confirmed that the rhodamine B and the methylene blue permeate into the gellan gum gel 38 in approximately equal amounts from both the positive-side microneedles 13 and the negative-side microneedles 14.

Next, mice are inoculated with a vaccine model (OVA) using the fluid injection device 10 shown in FIG. 7. In the test, the positive-side microneedle 13 and the negative-side microneedle 14 are immersed in a PBS buffer solution or an aqueous solution of 10 mg/mL OVA for 2 hours. That is, tests are conducted for three combinations of the positive-side microneedles 13 and the negative-side microneedles 14: i.e., PBS and PBS, OVA and PBS, and OVA and OVA. In the tests, a PBS aqueous solution is used as both the first fluid 21 on the positive-side microneedle 13 side and the second fluid 22 on the negative-side microneedle 14 side.

The tests are conducted as follows. First, as shown in FIG. 9(a), the positive-side microneedle 13 and the negative-side microneedle 14 of the fluid injection device 10 are pressed against the back of a hair-removed mouse 51, and a current is applied between the first electrode 24 and the second electrode 25 at a current density of 0.5 mA/cm² for one minute. After seven days, the current is again applied under the same conditions. After further seven days (14 days after the first electric current), the mouse 51 is euthanized, the blood is centrifuged to collect serum, and the IgG antibodies contained in the serum are analyzed. For measurement of antibody titer, 96-well plates are coated with 10 µg/mL of OVA overnight at 4°C and then blocked with 3% BSA. 100 µL of serum diluted with 1% BSA is added to the plate and incubated for two hours, after which biotin-labeled anti-mouse IgG antibody is added and incubated for further two hours, after which the absorbance at 450 nm is measured using a microplate reader. The measured absorbance is proportional to the amount of IgG antibody in the serum.

Results of the measurement are shown in FIG. 9 (b). Note that the measurement is carried out four times under conditions of each, and the error bars in a figure show standard deviation. Compared to a case where both the positive-side microneedle 13 and the negative-side microneedle 14 are PBS, when one is OVA, a greater absorbance (optical density) is observed, confirming a greater amount of IgG antibody in the serum. Moreover, it was confirmed that when both are OVA, the amount of IgG antibody produced is further greater. Moreover, for comparison, measurements are also taken when both are OVA and no current is applied (corresponding to the "No current" in the figure), and it is confirmed that almost no IgG antibody is produced. As a result, it is confirmed that OVA administration is achieved due to electroosmotic flow caused by current application.

### [Example of fluid injection device: Catheter-type device]

As shown in FIG. 10(a), a fluid injection device 10 may have a first transport body 11 and a second transport body 12, each of which may be made of a flexible tube 43 such as a catheter, and which are connected to each other on their outer surfaces along their length. In the example shown in FIG. 10( a ), there are one positive-side microneedle 13 and one negative-side microneedle 14. More specifically, the first transport body 11 and the second transport body 12 are each made of a silicone tube having an outer diameter of 2 mm.

As shown in FIG. 10(b), the fluid injection device 10 shown in FIG. 10(a) can easily access tissue inside the living body 1 and can directly inject a drug or the like into the tissue. This makes it possible to directly administer, for example, anticancer drugs to tumors, thereby providing effective treatment. In the past, for example, in Non-Patent Literature 2, which demonstrated the effect of iontophoresis on tumors, drug penetration from the positive electrode was utilized, and the negative electrode was placed far away, resulting in a design in which unnecessary current flowed throughout the body. In contrast, as shown in FIG. 10(b), the fluid injection device 10 shown in FIG. 10(a) can be inserted into the body of a living body 1 through a minimal incision using a catheter system, and can administer drugs directly to a tumor from both the anode and cathode without passing unnecessary current throughout the body.

### Reference Signs List

- 10: Fluid injection device
- 11: First transport body
21 First fluid
- 12: Second transport body
22 Second fluid
- 13: Positive-side microneedle
- 14: Negative-side microneedle
23 Flange portion
- 15: Current/voltage application means
24 First electrode
25 Second electrode
- 31: Chamber
- 32: Ag/Cl wire
- 33: Silicone rubber stopper
- 34: Source meters
- 35: Horizontal capillary
- 36: Pig skin slice
- 37, 38: Gellan gum gel
- 41: Tubular body
- 42a, 42b: Hollow portion
- 43: Tube
- 51: mouse

## Claims

1. A fluid injection device comprising:
a first transport body including a first transport flow path, a first fluid flowing through the first transport flow path;
a second transport body including a second transport flow path, a second fluid flowing through the second transport flow path;
a positive-side microneedle provided in the first transport body, the positive-side microneedle including a first opening at a tip thereof and a first flow path having a fixed positive charge, the first flow path communicating with the first opening, so that the first flow path communicates with the first transport flow path;
a negative-side microneedle provided in the second transport body, the negative-side microneedle including a second opening at a tip thereof and a second flow path having a fixed negative charge, the second flow path communicating with the second opening, so that the second flow path communicates with the second transport flow path; and
a current/voltage application means including a first electrode disposed in the first transport flow path and a second electrode disposed in the second transport flow path, the current/voltage application means capable of applying a current or voltage between the first electrode and the second electrode, wherein
when the current/voltage application means applies a current or voltage between the first electrode and the second electrode, an ionic current flows through the first fluid in the first flow path and the second fluid in the second flow path, and due to electroosmotic flow, the first fluid flows outward from the first opening and the second fluid flows outward from the second opening.

2. The fluid injection device according to claim 1, wherein the positive-side microneedle and the negative-side microneedle are disposed adjacent to each other.

3. The fluid injection device according to claim 1, wherein
the first transport body is elongated, the first transport flow path extends from one end to the other end,
the second transport body is elongated, the second transport flow path extends from one end to the other end,
the positive-side microneedle is provided at the one end of the first transport body, and
the negative-side microneedle is provided at the one end of the second transport body.

4. The fluid injection device according to claim 3, wherein
the positive-side microneedle is disposed so that its tip protrudes from the one end of the first transport body along an extension direction of the first transport body, and
the negative-side microneedle is disposed so that its tip protrudes from the one end of the second transport body along an extension direction of the second transport body.

5. The fluid injection device according to claim 1, wherein the first transport body and the second transport body are formed integrally.

6. The fluid injection device according to any one of claims 1-4, comprising
a rigid tubular body integrally forming the first transport body and the second transport body,
the tubular body is elongated and has two hollow portions therein extending from one end to the other, one of the hollow portions forms the first transport flow path, and the other hollow portion forms the second transport flow path.

7. The fluid injection device according to any one of claims 1-5, wherein the first transport body and the second transport body are each made of a flexible tube.
